Europäisches Patentamt

European Patent Office

Office européen des brevets

(19)

(11) Veröffentlichungsnummer: **0 003 023**

**B1**

(12) **EUROPÄISCHE PATENTSCHRIFT**

(21) Anmeldenummer: **78101574.8**

(22) Anmeldetag: **06.12.78**

(51) Int. Cl.³: **A 61 K 7/16**

(54) **Zahnpasta**

(30) Priorität: **22.12.77 DE 2757290**

(43) Veröffentlichungstag der Anmeldung:
**25.07.79 Patentblatt 79/15**

(45) Bekanntmachung des Hinweises auf die Patenterteilung:
**17.09.80 Patentblatt 80/19**

(84) Benannte Vertragsstaaten:
**BE CH DE FR GB IT NL SE**

(56) Entgegenhaltungen:
**FR - A - 930 740**
**FR - A - 1 047 979**
**FR - A - 2 211 211**
**GB - A - 1 476 063**
**US - A - 2 470 906**
**Chemical Abstracts, Vol. 81, Nr. 24, 16.**
**Dezember 1974, Columbus, Ohio, USA**
**Kato et al., "Zeolite as a polishing agent für**
**dentifrices. III. Properties of synthetic zeolite"**
**Seite 358, Auszug Nr. 158641s**

(73) Patentinhaber: **Blendax-Werke R. Schneider GmbH**
**& Co.**
**Rheinallee 88**
**D - 6500 Mainz (DE)**

(72) Erfinder: **Harth, Helmut, Dr.**
**Am Gonsenheimer Spiess 63**
**D - 6500 Mainz (DE)**
**Becker, Dieter**
**Messeler Parkstrasse 21**
**D - 6100 Darmstadt-Wixhausen (DE)**

Courier Press, Leamington Spa, England.

Zahnpasta

Die vorliegende Erfindung betrifft eine Zahnpasta, die gegenüber blanken Aluminiumoberflächen keine Korrosionswirkung aufweist und als alleiniges oder überwiegendes Poliermittel synthetischen Zeolith vom Alkalialuminiumsilikat-Typ enthält.

In einer Zahnpasta ist das Poliermittel neben Wasser und den Feuchthaltemitteln der quantitativ bedeutendste Bestandteil. Die bekanntesten in Zahnpasten eingesetzten Poliermittel sind dabei Calciumcarbonat, die verschiedenen Calciumphosphate wie insbesondere Dicalciumorthophosphat und Calciumpyrophosphat, unlösiches Alkalimetaphosphat, gefälltes Siliciumdioxyd-Gel, sei es in Form des Hydrogels oder des getrockneten Xerogels, wobei diese Putzkörper aufgrund ihrer Brechungsindices insbesondere bei der Herstellung transparenter und transluzenter Zahnpasten Verwendung gefunden haben, sowie Aluminiumhydroxid.

Die weiteren zahlreichen als Poliermittel in Zahnpasten vorgeschlagenen Substanzen haben im wesentlichen keine praktische Bedeutung erreicht, sondern stellen lediglich einen "papiernen Stand der Technik" dar.

Voraussetzung für die Eignung einer Substanz als Poliermittel in Zahnpasten ist, daß sie eine gewisse Abrasion, eben das Putzoder Poliervermögen, aufweist, ohne jedoch den Zahnschmelz zu schädigen. Mit anderen Worten: Das Mittel soll gerade so abrasiv sein, um den auf den menschlichen Zähnen vorhandenen Zahnbelag entfernen und die Interdentalräume reinigen zu können, ohne jedoch Zahnschmelz oder Dentin anzugreifen oder das Zahnfleisch übermäßig zu reizen.

Darüberhinaus soll das Poliermittel auch mit den heute in Zahnpasten eingesetzten Wirkstoffen verträglich sein, wobei besonderer Wert auf eine inerte Wirkung gegenüber den heute notorisch in Zahnpasten eingesetzten Fluorverbindungen wie Alkalifluoriden und Alkalimonofluorphosphaten gelegt wird.

Eine weitere wünschenswerte Eigenschaft für Poliermittel in Zahnpasten im besondern und Zahnpastenbestandteile im allgemeinen ist die, gegenüber blanken Aluminiumflächen nicht korrodierend zu wirken. Da zahlreiche Poliermittel jedoch gegenüber Aluminium korrosiv sind, ist es erforderlich, die zum Einsatz gelangenden Zahnpastentuben mit einem Innenschutzlack zu versehen, was natürlich die Kosten für das Verpackungsmaterial und damit auch für die fertige Zahnpasta erhöht und darüberhinaus bei eventuellen Schäden in der Innenschutzlackschicht trotzdem zu "Bombagen" der Zahnpastatuben und damit zu Reklamationen führen kann.

Es wurde nun gefunden, daß man ein nicht korrosives, mit Fluorverbindungen gut verträgliches, gut polierendes, jedoch nicht übermäßig abrasives Poliermittel erhält, wenn dieses Poliermittel zu mehr als 50% aus einem synthetischen Alkalialuminiumsilikat vom Zeolith-Typ A besteht.

Diese Produkte sind an sich bereits seit längerem bekannt, sie finden insbesondere als Molekularsiebe, zur Wasserenthärtung und neuerdings auch in Waschmitteln als Erstazstoffe für die sonst als anorganische Builder eingesetzten Phosphate Anwendung. Es handelt sich dabei um wasserhaltige Gerüstsilikate, die sich durch die allgemeine Formel $x(M \cdot AlO_2) \cdot ySiO_2 \cdot zH_2O$ ausdrücken lassen, wobei M Alkali oder Ammonium, x eine Zahl zwischen 1 und 64, y eine von x abhängige Zahl mit der Maßgabe, daß sie das ein- bis sechsfache von x, und z eine Zahl zwischen 0 und 256 bedeuten.

Die erfindungsgemäß als Poliermittel in Zahnpasten eingesetzten synthetischen Zeolithe vom Typ-A lassen sich auf einfache und an sich bekannte Weise aus Aluminiumhydroxyd und Alkalisilikaten, wie Wasserglas, herstellen. Ein im Rahmen der vorliegenden Erfindung besonders geeignetes Produkte ist ein Zeolith A mit der empirischen Formel $Na_{12}(AlO_2)_{12} \cdot (SiO_2)_{12} \cdot 27H_2O$. Ein solches Produkt wird von der Firma Degussa unter dem Handelsnamen "Sasil" vertrieben und weist eine mittlere Teilchengröße von etwa 4 $\mu$m, ein Schüttgewicht von etwa 400 g/l und einen Glühverlust von etwa 20% (1h bei 800°C), auf. Die zum Einsatz gelangenden Zeolithe sind, wie an sich nicht betont zu werden braucht, in Wasser unlöslich. Ihre durchschnittliche mittlere Teilchengröße liegt vorzugsweise zwischen etwa 1 und etwa 30 $\mu$m. Eine Übersicht über die Herstellung und die Eigenschaften der erfindungsgemäß zum Einsatz gelangenden Alkalialuminiumsilikate findet sich bei F. Schwochow und L. Puppe, Angewandte Chemie 87 (1975), S. 659 bis 667.

Aus der DE-OS Nr. 2 146 224 sind zwar bereits transparente bzw. transluzente Zahnpasten bekannt, die als Putzkörper ein synthetisches amorphes komplexes Alkali- oder Erdalkalialuminiumsilikat enthalten, das einen Brechunsindex von etwa 1,44 bis 1,47 aufweist. Die dort beschriebenen Alkalialuminiumsilikate unterscheiden sich jedoch wesentlich von den anmeldungsgemäß zum Einstaz gelangenden Produkten, da sie keine synthetischen Zeolithe darstellen; darüberhinaus ist der Gegenstand dieser DE-OS hinsichtlich Aufgabenstellung (Herstellung transparenter Zahnpasten) von dem erfindungsgemäßen Vorschlag (antikorrosives Poliermittel mit optimalem Poliervermögen und Fluorverträglichkeit) völlig unterschiedlich. Auch sind die erfindungsgemäßen Zahnpasten nicht transparent bzw. transluzent.

Die GB-PS Nr. 332 142 beschreibt Zahnpulver, die Zeolith nicht näher definierter Zusammensetzung als Calciumionen-Komplexbildner enthalten. Von korrosionsverhindernden oder polierenden Eigenschaften dieses Zeoliths in Zahnpastatuben ist in dieser Veröffentlichung hingegen keine Rede, da bei Zahnpulvern Korrosionsprobleme nicht auftreten.

Ähnliches gilt hinsichtlich der Offenbarung der DE-PS Nr. 378 010, wo die dort zum Einsatz in Zahnreinigungsmitteln vorgeschlagenen Zeolithe unbekannter Herkunft und Zusammensetzung ebenfalls aufgrund ihrer komplexierenden Wirkung auf Erdalkaliionen zum Einsatz gelangen.

In Chemical Abstracts, Vol. 81 (1974), No. 158641s, ist eine japanische Arbeit referiert, in der angeregt wird, einen Zeolith vom Faujasit-Typ als Poliermittel in Zahnpflegemitteln einzusetzen. Es wird jedoch zugleich festgestellt, daß dieser Faujasit im Gegensatz zum erfindungsgemäß zum Einsatz gelangenden Zeolith vom A-Typ Fluor absorbiert, so daß er zum Einsatz in fluorhaltigen Zahnpasten, die gegenwärtig auf dem Markt dominieren, nicht als geeignet erscheinen mußte. Es war daher überraschend, daß der erfindungsgemäß zum Einsatz gelangende, genau definierte Zeolith A sich gegenüber Fluorverbindungen völlig inert verhält, also kienerlei Absorption oder sonstige Inaktivierung auftritt. Darüberhinaus läßt sich dieser Veröffentlichung nichts über die überraschenderweise vom pH-Wert unabhängige, korrosionsstabilisierende Wirkung des erfindungsgemäßen Poliermittels in wäßrigen Zahnpasten entnehmen.

Mit der Aufgabenstellung und erst recht der Aufgabenlösung nach der vorliegenden Erfindung stehen diese Druckschriften also in keinem engeren Zusammenhang.

Die Menge an Poliermittel beträgt in Zahnpasten üblicherweise mindestens 15 und maximal 60 Gew.-% und liegt in der Regel vorzugsweise zwischen etwa 25 und etwa 40 Gew.-%.

Obwohl erfindungsgemäß die alleinige Anwendung des Alkalialuminiumsilikat-Poliermittels vom Zeolith A-Typ bevorzugt ist, ist es prinzipiell möglich, diesen Poliermitteln auch noch untergeordnete Mengen weitere an sich bekannter Poliermittel zuzusetzen, ohne daß die positiven Eigenschaften des Zeoliths beeinträchtigt werden, vorausgesetzt, dessen Anteil liegt bei mehr als 50 Gew.-% des Poliermittelgemisches. Poliermittel, die in untergeordneten Mengen gemeinsam mit dem Zeolith Einsatz finden können, sind beispielsweise die verschiedenen Calciumphosphate wie Dicalciumphosphat in seiner wasserfreien oder hydrathaltigen Form, Calciumpyrophosphat und Tricalciumphosphat, Silicagele wie Siliciumdioxyd-Hydrogel oder Siliciumdioxyd-Xerogele, wie sie insbesondere von der Firma Grace GmbH unter dem Namen "Syloid" vertrieben werden, Calciumcarbonat, Aluminiumhydroxyd, feinverteilte pulverförmige Kunststoffe oder unlösliches Alkalimetaphosphat.

Als Feuchthaltemittel finden Glycerin, Polyglykole mit niederem Molgewicht oder Zuckeralkohole wie Sorbit, Mannit und Xylit Verwendung.

Zahnpasten enthalten ferner Verdickungsmittel. Als solche eignen sich am besten die Alkalisalze der Carboxymethylcellulose, insbesondere Natriumcarboxymethylcellulose, Hydroxyalkylcellulosen, insbesondere Hydroxyäthylcellulose, Pflanzengummen wie Traganth, Gummi arabicum, Caraya-Gummi und Irish Moos, synthetische Polyelektrolyte wie das Natrium-, Kalium- oder Ammoniumsalz der Polyacrylsäure und gegenbenenfalls auch anorganischer Verdickungsmittel, beispielsweise kolloidales Magnesiumaluminiumsilikat.

Der Anteil des Verdickungsmittels beträgt etwa 0,25 bis 5 Gew.-% der Zahnpasta.

Weiterer häufiger Bestandteil von Zahnpasten sind oberflächenaktive Substanzen.

Als solche eignen sich besonders wasserlösliche Salze von höheren Alkylsulfaten, beispielsweise Natriumlaurylsulfat, aliphatische Acylamide gesättigter Monoaminocarbonsäuren, vorzugsweise Natrium-N-lauroylsarcosinat, Taurin-Fettsäureamide, beispielsweise Natrium-N-alkyl-N-myristoyltaurid, Salze von sulfonierten Monoglyceriden höherer Fettsäuren, beispielsweise Natriummonoglyceridsulfonat, Fettsäureester der Isäthionsäure und deren Salze, nichtionische Tenside wie Alkylenoxidkondensate mit Fettalkoholen und ein- oder mehrwertigen Aminen, Zuckerester, beispielsweise Saccharosemonolaurat, Sorbitpolyoxyäthylenstearat, langkettige Amin-oxide, beispielsweise Dimethyllaurylaminoxid, ampholytische Tenside, beispielsweise Betaine oder langkettige Alkylaminocarbonsäuren und kationaktive Tenside, beispielsweise quartäre Ammoniumverbindungen wie Cetyltrimethylammoniumbromid.

Der Anteil an oberflächenaktiven Verbindungen in dem erfindungsgemäßen Zahnpflegemittel liegt bei 0 bis etwa 5 Gew.-% der Gesamtzusammensetzung.

Zahnpflegemittel enthalten üblicherweise Aroma- und Geschmacksstoffe, Konservierungsmittel etc., derartige Mittel sind an sich bekannt und in zahlreichen Druckschriften beschrieben.

Wie bereits angedeutet, weisen die im erfindungsgemäßen Zahnpflegemittel zum Einsatz gelangenden Poliermittel auf Basis eines synthetischen Zeoliths vom A-Typ eine ausgezeichnete Verträglichkeit mit ionischem oder komplex gebundenem Fluor auf. Es ist daher eine bevorzugte Ausführungsform der Erfindung, solche Fluorverbindungen in dem erfindungsgemäßen Zahnpflegemittel mitzuverwenden, vorzugsweise in solchen Mengen, daß die Konzentration an reinem Fluor im Mittel etwa 0,01 bis etwa 1 Gew.-%, vorzugsweise 0,1 bis 0,5 Gew.-%, des Zahnpflegemittels beträgt.

Geeignete Fluorverbindungen sind insbesondere die verschiedenen Salze der Monofluorphosphorsäure, insbesondere Natrium-, Kalium-, Lithium-, Calcium- und Aluminiummono- und difluorphosphat, sowie die verschiedenen, Fluor in ionisch gebundener Form enthaltenden Fluoride, insbesondere Alkalifluoride wie Natrium-, Lithium-, Kalium- und Ammoniumfluorid, Zinnfluorid, Manganfluorid, Zirkoniumfluorid und Aluminiumfluorid sowie Gemische oder Anlagerungsprodukte dieser Fluoride untereinander oder mit anderen Fluorverbindungen, beispielsweise Kalium- oder Natriummanganfluorid.

Andere im Rahmen der vorliegenden Erfindung einsetzbare Fluoride sind beispielsweise Zinkfluorid, Germaniumfluorid, Palladiumfluorid, Titanfluorid, Alkalifluorzirkonate, beispielsweise Natrium- oder Kaliumfluorzirkonat, Zinnfluorzirkonat, Fluorborate oder Fluorsulfate, beispielsweise Natrium- oder Kaliumfluorosulfat.

Auch organische Fluorverbindungen können mit Erfolg eingesetzt werden, insbesondere die bekannten Additionsprodukte aus langkettigen Aminen oder Aminosäuren und Fluorwasserstoff, Monoäthanolaminohydro-fluorid oder Methyltriäthylammoniumfluorid.

Die erfindungsgemäßen Zahnpflegemittel können weitere, zur Verwendung in solchen Mitteln an sich bekannte Stoffe enthalten, beispielsweise Enzyme wie Proteasen und Carbohydrasen wie Amylase, Dextranase, Lävanase oder α-1,3-Glucan-3-glucanohydrolase, Zahnbelag entfernende Substanzen wie die für diesen Zweck vorgeschlagenen Phosphonsäuren, beispielsweise Hydroxyäthan-1,1-diphosphonsäure, oder die unter den Namen "Chlorhexidin", "Fluorhexidin" oder "Alexidin" bekannten Bisbiguanide 1,6-Di-4'-(chlorphenyldiguanido)hexan, 1,6-Di-4'-(fluorphenyldiguanido)hexan und 1,6-Di-(2-äthylhexyldiguanido)hexan bzw. deren vorzugsweise wasserlöslichen Salze.

Eine ausführliche Übersicht über die Herstellung von Zahnpflegemitteln und die dabei zum Einsatz gelangenden Stoffe findet sich in dem Handbuch von M. S. Balsam und E. Sagarin. "Cosmetics—Science and Technology", 2nd Ed., Vol. 1, S. 423 bis 532 (1972).

Im folgenden werden einige Beispiele für erfindungsgemäß zusammengesetzte Zahnpflegemittel gegeben:

### Beispiel 1

| | |
|---|---|
| Carboxymethylcellulose | 1,30 Gew.-% |
| p-Hydroxybenzoesäuremethylester | 0,10 Gew.-% |
| Glycerin | 20,00 Gew.-% |
| Aroma | 1,00 Gew.-% |
| 1,2-Propylenglykol | 2,00 Gew.-% |
| Benzoesäure | 0,50 Gew.-% |
| Saccharin-Natrium | 0,05 Gew.-% |
| Alkalimonofluorphosphat | 0,75 Gew.-% |
| Natriumlaurylsulfat | 1,80 Gew.-% |
| Natriumaluminiumsilikat (Zeolith A, $Na_{12}(AlO_2)_{12}(SiO_2)_{12} \cdot 27H_2O$) | 35,000 Gew.-% |
| pyrogene Kieselsäure | 2,50 Gew.-% |
| Titandioxid | 0,70 Gew.-% |
| Wasser | 34,30 Gew.-% |

Das Natriumaluminiumsilikat kann in den beispielhaft angegebenen Rezepturen ganz oder teilweise jeweils auch durch die entsprechende Kalium- oder Lithium- verbindung ersetzt werden.

Die Teilchengrößen der erfindungsgemäß zum Einsatz gelangenden Zeolithe liegen im für Zahnpasten-Poliermittel üblichen Bereich zwischen etwa 1 bis etwa 20 Mikron, vorzugsweise zwischen 2 und 10 Mikron.

### Beispiel 2

| | |
|---|---|
| Hydroxyäthylcellulose | 1,20 Gew.-% |
| p-Hydroxybenzoesäureester | 0,08 Gew.-% |
| Sorbit, 70 %ig | 7,00 Gew.-% |
| Benzoesäure | 0,30 Gew.-% |
| 1,2-Propylenglykol | 3,00 Gew.-% |
| Aroma | 1,00 Gew.-% |
| Saccharin-Natrium | 0,04 Gew.-% |
| mediz. Seife | 0,60 Gew.-% |
| pyrogene Kieselsäure | 0,20 Gew.-% |
| Alkalimonofluorphosphat | 0,75 Gew.-% |
| Natriumaluminiumsilikat (Zeolith A, $Na_{12}(AlO_2)_{12}(SiO_2)_{12} \cdot 27H_2O$) | 25,00 Gew.-% |
| Calciumcarbonat | 20,000 Gew.-% |
| Wasser | 40,83 Gew.-% |

### Beispiel 3

| | |
|---|---|
| Carragheenat | 0,70 Gew.-% |
| para-Hydroxybenzoesäurepropylester | 0,02 Gew.-% |

| para-Hydroxybenzoesäuremethylester | 0,05 Gew.-% |
| --- | --- |
| Benzoesäure | 0,20 Gew.-% |
| Glycerin | 18,00 Gew.-% |
| 1,2-Propylenglykol | 2,00 Gew.-% |
| Aroma | 1,00 Gew.-% |
| Saccharin-Natrium | 0,04 Gew.-% |
| Alkalimonofluorphosphat | 0,75 Gew.-% |
| pyrogene Kieselsäure | 1,50 Gew.-% |
| unlösl. Na-Metaphosphat | 20,00 Gew.-% |
| Natriumaluminiumsilikat (Zeolith A, $Na_2O \cdot Al_2O_3 \cdot 2SiO_2 \cdot 4,5H_2O$) | 25,00 Gew.-% |
| Natriumlauroylsarcosinat | 1,50 Gew.-% |
| Wasser | 29,24 Gew.-% |

### Beispiel 4

| Carboxymethylcellulose | 1,20 Gew.-% |
| --- | --- |
| p-Hydroxybenzoesäuremethylester | 0,07 Gew.-% |
| Benzoesäure | 0,20 Gew.-% |
| Sorbit, 70 %ig | 18,00 Gew.-% |
| Glycerin | 6,00 Gew.-% |
| Aroma | 1,00 Gew.-% |
| Saccharin-Natrium | 0,05 Gew.-% |
| Alkalimonofluorphosphat | 0,75 Gew.-% |
| Dicalciumphosphat ($CaHPO_4$) | 10,00 Gew.-% |
| Natriumaluminiumsilikat (Zeolith A, $Na_{12}(AlO_2)_{12}(SiO_2)_{12} \cdot 27H_2O$) | 28,00 Gew.-% |
| Natriumlaurylsulfat | 2,00 Gew.-% |
| pyrogene Kieselsäure | 2,00 Gew.-% |
| Farbstoff | 0,03 Gew.-% |
| Wasser | 30,00 Gew.-% |

## Patentansprüche

1. Zahnpasta auf wäßriger Basis, enthaltend ein Poliermittel und übliche Bestandteile, dadurch gekennzeichnet, daß das Poliermittel zum überwiegenden Teil aus einem synthetischen Alkalialuminiumsilikat vom Zeolith-Typ A der allgemeinen Formel $x(M \cdot AlO_2) \cdot ySiO_2 \cdot zH_2O$, wobei M Alkali oder Ammonium, x eine Zahl zwischen 1 und 64, y eine von x abhängige Zahl mit der Maßgabe, daß sie das ein- bis sechsfache von x bedeutet, und z eine Zahl zwischen 0 und 256 bedeuten, besteht.

2. Zahnpasta nach Anspruch 1, dadurch gekennzeichnet, daß sie das Alkalialuminiumsilikat vom Zeolith-Typ A als alleiniges Poliermittel enthält.

3. Zahnpasta nach Anspruch 1 und/oder 2, dadurch gekennzeichnet, daß sie als Alkalialuminiumsilikat von Zeolith-Typ A ein Natriumaluminiumsilikat enthält.

4. Zahnpasta nach Anspruch 3, dadurch gekennzeichnet, daß sie ein Natriumaluminiumsilikat vom Zeolith-Typ A der empirischen Formel $Na_{12}(AlO_2)_{12}(SiO_2)_{12} \cdot 27H_2O$ enthält.

## Claims

1. Aqueous toothpaste composition, containing a polishing agent and usual compounds, whereby the major portion of said polishing agent is composed of a synthetical alkali aluminium silicate of zeolite-type A of the general formula $x(M \cdot AlO_2) \cdot ySiO_2 \cdot zH_2O$, wherein M is alkali or ammonium, x is a number from 1 to 64, y is a number dependent on x with the determination of y being one-fold to six-fold of x, and z is a number from 0 to 256.

2. Toothpaste composition according to claim 1, containing the alkali aluminium silicate of zeolite-type A as the sole polishing agent.

3. Toothpaste composition according to claim 1 and/or 2, containing as alkali aluminium silicate of zeolite-type A a sodium alkali aluminium silicate.

4. Toothpaste composition according to claim 3, containing a sodium aluminium silicate of zeolite-type A of the empirical formula $Na_{12}(AlO_2)_{12}(SiO_2)_{12} \cdot 27H_2O$.

## Revendications

1. Dentifrice sur base aqueuse, contenant un agent de polissage et les composants usuels, caractérisé par le fait que l'agent de polissage se compose d'une proportion majeure d'un silicate d'aluminium alcalin synthétique d'un type zéolithe A de la formule générale $x(M \cdot AlO_2) \cdot ySiO_2 \cdot zH_2O$ dont M signifie alcali ou ammonium, x un nombre entre 1 et 64, y un nombre dépendant de x avec la mesure, qu'il signifie le simple jusqu'au sextuple de x et dont z signifie un nombre entre 0 et 256.

2. Dentifrice selon revendication 1, caractérisé par le fait qu'il contient le silicate d'aluminium alcalin du type zéolithe A comme seule agent de polissage.

3. Dentifrice selon revendication 1 et/ou 2, caractérisé par le fait qu'il contient un silicate d'aluminium de sodium comme silicate d'aluminium alcalin du type zéolithe A.

4. Dentifrice selon revendication 3, caractérisé par le fait qu'il contient un silicate d'aluminium de sodium du type zéolithe A de la formule empirique $Na_{12}(AlO_2)_{12}(SiO_2)_{12} \cdot 27H_2O$.